# DEMANDE DE BREVET EUROPEEN

(11) **EP 1 997 462 A1**
(43) Date de publication de la demande: **03.12.2008**
(21) Numéro de dépôt: 07360023.1
(22) Date de dépôt: 01.06.2007
(51) Int. Cl.: A61F 13/12, A61F 13/20, A61F 13/36

(54) **Pansement intrabuccal**

(71) Demandeur: Lesca, Christophe, 75016 Paris (FR)
(72) Inventeur: Lesca, Christophe, 75016 Paris (FR)
(74) Mandataire: Littolff, Denis

(57) **Abrégé**

Pansement utilisable en chirurgie intrabuccale, consistant en l'association d'une toile souple stérilisable à une colle chimique durcissant au contact de l'air.

Cette colle est visqueuse. La toile en est imprégnée et enduite sous vide. L'ensemble est conditionné sous vide.

## Description

La présente invention a trait à un pansement utilisable en chirurgie intrabuccale, associant notamment une toile stérilisable à une colle chimique durcissant au contact de l'air.

Ce nouveau pansement s'adresse prioritairement aux patients sous anticoagulants, ainsi qu'à ceux qui présentent un déficit modéré en facteur de coagulation, soit congénital soit acquis. Plus précisément, il est par exemple indiqué pour des personnes qui suivent des traitements intégrant des anti-vitamines K, de l'héparine ou des anti-agrégants plaquettaires. Il est par ailleurs recommandé lors d'opérations concernant des individus par exemple atteints d'hémophilie A et B ou de maladie de Willebrand, et dont le sang ne coagule par conséquent pas correctement pour des raisons congénitales. Le même problème peut d'ailleurs se poser pour des personnes par exemple en insuffisance hépato-cellulaire, affection qui concerne notamment les cirrhotiques.

Si le pansement de l'invention est utilisé essentiellement pour des actes chirurgicaux relevant de la sphère intrabuccale, et à titre principal pour les extractions dentaires, d'autres actes de la sphère ORL peuvent néanmoins être concernés.

En cas d'extraction dentaire chez des patients présentant un déficit du facteur de coagulation ou sous anticoagulant, l'hémorragie post-opératoire peut nécessiter une reprise chirurgicale en urgence. C'est pour éviter ce problème que l'invention a été conçue, proposant un pansement qui rend le site de l'opération hermétique à toute suffusion hémorragique pendant tout le temps de sa présence.

A cet effet, le pansement de l'invention, associant comme indiqué auparavant une toile souple stérilisable à une colle chimique durcissant au contact de l'air, se caractérise également en ce que :
- ladite colle est visqueuse ;
- la toile en est imprégnée et enduite sous vide ; et
- l'ensemble est conditionné sous vide.
   Ce pansement permet de réaliser un coffrage moulant et débordant la surface opérée, empêchant toute suffusion. Il est de préférence suturé à la gencive avec du fil résorbable, au niveau des berges alvéolaires après extraction de la dent.
   Selon une possibilité préférentielle, la toile est une maille absorbante d'environ 1 mm d'épaisseur, dont la souplesse permet une adaptation à la morphologie particulière des berges alvéolaires après extraction de la dent. De préférence encore, cette toile peut être de la gaze de cellulose.
   Dans l'hypothèse, également préférentielle, où cette toile souple est résorbable, et si la suture est pratiquée avec un fil résorbable, le pansement rendant le site opératoire hermétique se délite spontanément entre le troisième et le dixième jour post-opératoire. Il est cependant évidemment aussi possible d'utiliser une toile non résorbable avec un fil, résorbable ou non, respectivement provoquant un décollement de la toile entre le 10^{e} et le 20^{e} jour et nécessitant une opération manuelle pour retirer la toile. Dans l'hypothèse encore différente où la toile est résorbable et pas le fil, ladite toile se délite dans le délai susmentionné, et il faut évidemment enlever le fil manuellement.
   Selon une possibilité, la colle utilisée est de type cyanoacrylate, qui durcit à l'air en quelques minutes. En fait, le durcissement résulte d'une polymérisation provoquée par la vapeur d'eau contenue dans l'air, et par l'hydratation de la muqueuse buccale une fois le pansement positionné.
   Lorsque le pansement est mis en place sur le site opératoire, il en épouse le relief avant séchage de la colle cyanoacrylate. Dès le durcissement qui accompagne le séchage à l'air, le coffrage moulant et débordant la surface opérée est opérationnel, permettant d'éviter le risque d'hémorragie.
   Les bénéfices principaux attendus de ce type de pansements sont les suivants
- protection temporaire du caillot sanguin du milieu intrabuccal ; et
- création d'une barrière rigide inextensible.

Il est à noter que les points de suture maintenant le pansement provoquent une diminution de la pression de perfusion des berges alvéolaires, par un effet de strangulation de la muqueuse.

La pose du pansement se réalise en outre en dehors de toute hospitalisation et de toute modification des traitements anticoagulants pris par les patients. En fait, plus précisément, après l'extraction dentaire, il est procédé à un curetage alvéolaire soigneux. Ensuite, le chirurgien met en place un matériau hémostatique de type gaze de cellulose non tassé dans l'alvéole et procède à la suture des berges muqueuses péri-alvéolaires. Le site opératoire est ensuite asséché par compression, afin d'éliminer tout liquide biologique.

Le site étant ainsi préparé, le chirurgien déconditionne le pansement pré-encollé de l'invention et le découpe à la morphologie de la crête alvéolaire, de manière à déborder l'alvéole et d'éventuelles incisions chirurgicales sur un à deux centimètres. Le pansement est ensuite mis en place sur le site, afin qu'il en épouse le relief, et il y est maintenu jusqu'à durcissement de la colle. Le chirurgien procède ensuite à la suture, de préférence avec du fil résorbable et au moins aux quatre points cardinaux. Enfin, le pansement recouvrant ainsi de manière hermétique le site opératoire est badigeonné à l'aide d'un bâton de colle cyanoacrylate, afin d'avoir un champ exsangue en post-opératoire immédiat.

Alternativement, il est possible de démarrer la suture dès le positionnement du pansement sur le site à protéger, l'aiguille traversant alors plus facilement le pansement imprégné de colle encore visqueuse, laquelle peut au surplus colmater les points de poncture de la muqueuse liés au passage de l'aiguille et du fil.

L'invention telle que décrite n'est pas limitée aux exemples fournis. Ainsi, le matériau de la toile souple n'est pas limité à la gaze de cellulose, et le type de colle mentionné n'est pas exhaustif non plus.

## Revendications

1. Pansement utilisable en chirurgie intrabuccale, **caractérisé en ce qu'**il consiste en l'association d'une toile souple stérilisable à une colle chimique durcissant au contact de l'air, et **en ce que** :
- ladite colle est visqueuse ;
- la toile en est imprégnée et enduite sous vide ; et
- l'ensemble est conditionné sous vide.

2. Pansement selon la revendication précédente, **caractérisé en ce que** la toile est une maille absorbante d'environ 1 mm d'épaisseur.

3. Pansement selon la revendication précédente, **caractérisé en ce que** la toile est une gaze de cellulose.

4. Pansement selon l'une quelconque des revendications précédentes, **caractérisé en ce que** la toile souple est résorbable.

5. Pansement selon l'une quelconque des revendications précédentes, **caractérisé en ce que** la colle est de type cyanoacrylate.
